(19) Europäisches Patentamt
European Patent Office
Office européen des brevets

(11) Veröffentlichungsnummer: **0 085 158**
**B1**

(12) # EUROPÄISCHE PATENTSCHRIFT

(45) Veröffentlichungstag der Patentschrift: **19.06.85**

(21) Anmeldenummer: **82111453.5**

(22) Anmeldetag: **10.12.82**

(51) Int. Cl.⁴: **C 07 C 49/707**, C 07 C 49/713, C 07 C 45/64, C 07 C 45/65, C 25 B 3/04

(54) **Verfahren zur Herstellung von Cycloalkenonderivaten.**

(30) Priorität: **28.01.82 CH 520/82**

(43) Veröffentlichungstag der Anmeldung: **10.08.83 Patentblatt 83/32**

(45) Bekanntmachung des Hinweises auf die Patenterteilung: **19.06.85 Patentblatt 85/25**

(84) Benannte Vertragsstaaten: **AT BE CH DE FR GB IT LI NL**

(56) Entgegenhaltungen:
**EP - A - 0 005 748**
**EP - A - 0 031 875**
**FR - A - 956 392**
**FR - A - 1 438 234**
**US - A - 3 444 206**

**J. ELECTROCHEM. SOC.: ELECTROCHEMICAL SCIENCE, Band 117, Nr. 3, März 1970, Seiten 342-343**

(73) Patentinhaber: **F. HOFFMANN-LA ROCHE & CO. Aktiengesellschaft, CH-4002 Basel (CH)**

(72) Erfinder: **Grass, Hansjörg, Dr., Wiesengrundstrasse 7, CH-4132 Muttenz (CH)**
Erfinder: **Widmer, Erich, Dr., Mittelweg 47, CH-4142 Münchenstein (CH)**

(74) Vertreter: **Cottong, Norbert A. et al, *Grenzacherstrasse 124 Postfach 3255, CH-4002 Basel (CH)***

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 085 158 B1

**Beschreibung**

Die vorliegende Erfindung betrifft die Herstellung von Zwischenprodukten in Carotinoidsynthesen, nämlich die Herstellung von 3-Hydroxy-2-cycloalken-1-on-Derivaten.

Das erfindungsgemäße Verfahren ist dadurch gekennzeichnet, daß man eine Verbindung der allgemeinen Formel

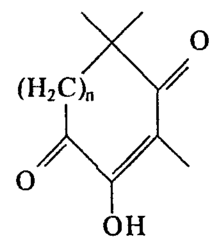

(I)

worin n die Zahlen 0 oder 1 bedeutet,
zu einer Verbindung der allgemeinen Formel

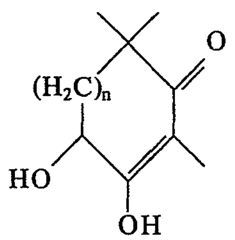

(II)

worin n die obige Bedeutung hat,
kathodisch reduziert und, gewünschtenfalls, die erhaltene Verbindung in saurem oder neutralem Medium zu einer Verbindung der allgemeinen Formel

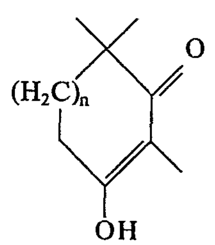

(III)

worin n die obige Bedeutung hat,
kathodisch weiterreduziert.

Aus der Europäischen Patentpublikation Nr. 0 031 875 ist die Reduktion der Verbindung der obigen Formel I, worin n 1 ist, zur entsprechenden Verbindung der Formel III mittels Zink und einer Carbonsäure bekannt. Dieses Verfahren hat jedoch den Nachteil, daß mit einem Überschuß an Zink gearbeitet werden muß und daher große Mengen an Zinksalzen anfallen.

Es wurde nun gefunden, daß das erfindungsgemäße Verfahren nicht mit den Nachteilen des vorbekannten Verfahrens verbunden ist und dennoch ermöglicht, die gewünschten Produkte in guter Ausbeute zu erhalten. Da die zur Reduktion der Verbindungen der Formeln I und II erforderlichen Potentiale genügend verschieden sind, können nach diesem Verfahren selektiv entweder Verbindungen der Formel II oder Verbindungen der Formel III erhalten werden. Das erfindungsgemäße Verfahren ist zudem leicht als kontinuierliches Verfahren durchführbar.

Die Verbindung der obigen Formel II, worin n Null ist, d. h. die Verbindung der Formel

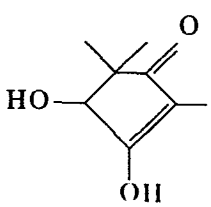

(IIa)

ist neu und bildet ebenfalls Gegenstand der vorliegenden Erfindung.

Die Verbindungen der obigen Formeln II und III können in tautomeren Formen vorliegen und werden

deshalb nach dem erfindungsgemäßen Verfahren als entsprechendes Tautomerengemisch erhalten:

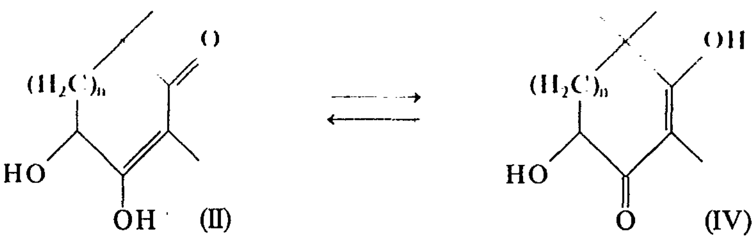

(II) (IV)

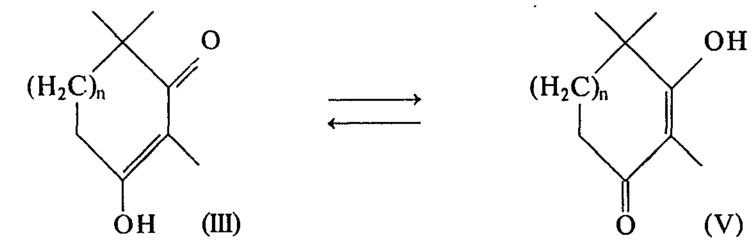

(III) (V)

In der Literatur wird für solche Verbindungen gelegentlich auch das tautomere 1,3-Diketon angegeben. Es hat sich jedoch gezeigt, daß die Verbindungen der Formeln II und III praktisch ausschließlich in den obigen Ketonol-Formen vorliegen.

Das erfindungsgemäße Verfahren kann in ungeteilten oder unterteilten Zellen durchgeführt werden. Vorzugsweise wird jedoch eine unterteilte Zelle verwendet, wobei die Unterteilung mit Membranen oder Diaphragmen aus üblichen Membran- bzw. Diaphragma-Materialien, beispielsweise Ton, Keramik, Glas (z. B. Glassinterdiaphragma) oder polymeren Verbindungen (z. B. NAFION® [Dupont]) erfolgen kann.

Die Elektroden können übliche Formen aufweisen. Beispielsweise können die Elektroden in Form von Platten oder Gittern oder als Streckmetall ausgebildet sein.

Bevorzugte Kathodenmaterialien sind Quecksilber (beispielsweise als Quecksilbersumpfelektrode), Blei und Graphit. Je nach Reaktionsbedingungen kommen jedoch auch weitere Kathodenmaterialien in Frage, beispielsweise in stark sauren Lösungen Cadmium, Zinn, Aluminium oder mit Blei beschichtete Stahl- oder Kupfernetze, in schwach sauren oder neutralen Lösungen Cadmium, Zink oder Zinn und in basischen Lösungen Nickel. Ferner können für die Reduktion einer Verbindung der Formel I zur entsprechenden Verbindung der Formel II in saurer Lösung auch Nickel, Kupfer, Stahl, Vanadium, Silber, Kobalt, Messing und dergleichen verwendet werden.

Die beim erfindungsgemäßen Verfahren verwendeten Anodenmaterialien sind nicht kritisch. Geeignete Anodenmaterialien sind beispielsweise Platin, Palladium, Silber, Gold, Graphit oder Blei. Ferner können auch dimensionsstabile Anoden [wie z. B. in A. Schmidt, Angewandte Elektrochemie, S. 70, Verlag Chemie (1976) erwähnt], auch Metalloxid-Verbundanoden genannt, verwendet werden. Solche Anoden bestehen aus einem Träger aus Titan, Eisen, Nickel und dergleichen, welcher mit einer Metalloxidauflage (z. B. Bleidioxid, Mangandioxid, Rutheniumdioxid oder Titansuboxid) versehen ist, wobei auf der Metalloberfläche vor der Aufbringung des Metalloxidüberzuges eine Zwischenschicht aus einem Carbid oder Borid der Elemente der IV. und V. Nebengruppe aufgebracht wird.

Die erfindungsgemäße Elektrolyse wird bei Verwendung eines sauren oder neutralen Reaktionsmediums zweckmäßig mit einem Gemisch eines Elektrolyten, eines inerten organischen Lösungsmittels (Kolösungsmittel) und der als Ausgangsmaterial verwendeten Verbindung der Formel I durchgeführt.

Als Elektrolyten können beispielsweise wäßrige Säuren, wie Schwefelsäure, Flußsäure, Salzsäure, Phosphorsäure, Perchlorsäure, wäßrige Trifluoressigsäure und dergleichen verwendet werden. Als besonders geeignet haben sich hierbei Mineralsäuren, insbesondere die Schwefelsäure erwiesen. Zweckmäßigerweise arbeitet man mit etwa 0,1N bis etwa 18N, vorzugsweise etwa 1N bis etwa 10N wäßrigen Säuren.

Als Kolösungsmittel kommen wassermischbare oder nicht wassermischbare inerte organische Lösungsmittel in Frage. Bei Verwendung eines nicht wassermischbaren Kolösungsmittels, wie beispielsweise 1,2-Dichloräthan oder Tetrachlorkohlenstoff, wird zweckmäßig ein Phasentransferkatalysator (vorzugsweise ein Tetraalkylammoniumsalz z. B. Tetrabutylammonium-hydrogensulfat) zugesetzt.

Vorzugsweise wird jedoch ein wassermischbares Kolösungsmittel verwendet, beispielsweise ein Alkohol, wie Methanol, Äthanol oder t-Butanol, ein cyclischer Äther, wie Tetrahydrofuran oder 1,4-Dioxan, ein Nitril, wie Acetonitril, ein Amid, wie N,N-Dimethylformamid oder Hexamethylphosphorsäuretriamid, eine Carbonsäure, wie Ameisensäure oder Essigsäure, oder Propylencarbonat, Dimethylsulfoxid oder Aceton. Bevorzugte derartige Kolösungsmittel sind Methanol, Äthanol, t-Butanol, Tetrahydrofuran und 1,4-Dioxan.

Bei Verwendung einer Carbonsäure als Kolösungsmittel kann als Elektrolyt beispielsweise auch eine wäßrige Lösung eines Carboxylates (z. B. Natriumformiat, Natriumacetat) eingesetzt werden.

Anderseits ist es auch möglich, eine wäßrige Lösung einer Carbonsäure, wie Ameisensäure, als Elektrolyt zu verwenden. Hierbei wird zur Verbesserung der Leitfähigkeit zweckmäßig ein Leitsalz (z. B. Natriumformiat) zugesetzt und als Kolösungsmittel beispielsweise ein Alkohol, wie Methanol, verwendet.

Das Volumenverhältnis Elektrolyt/Kolösungsmittel beträgt bei Verwendung eines wassermischbaren Kolösungsmittels zweckmäßig etwa 5 : 1 bis etwa 1 : 5 und vorzugsweise etwa 1 : 1. Bei Verwendung eines nicht wassermischbaren Kolösungsmittels wird hingegen im allgemeinen nur soviel Kolösungsmittel zugesetzt, wie notwendig ist, um eine ausreichende Löslichkeit des Edukts und des Produkts zu gewährleisten.

Weiterhin kann als Elektrolyt auch eine wäßrige Lösung einer Base, beispielsweise etwa 0,1N—5N Natronlauge oder Kalilauge, verwendet werden. Da Edukt und Produkt in solchen Elektrolyten ausreichend löslich sind, erübrigt sich die Verwendung eines Kolösungsmittels. Die zur Reduktion erforderlichen Potentiale sind jedoch in basischen Reaktionsmedien negativer als in sauren oder neutralen Medien. Während die Reduktion der Verbindungen der Formel I zu Verbindungen der Formel II in basischen Lösungen noch problemlos möglich ist, gelingt es im allgemeinen nicht mehr, die Verbindungen der Formel II zu Verbindungen der Formel III weiter zu reduzieren, da bei den hierzu erforderlichen negativen Potentialen bereits Zersetzung des Lösungsmittels eintritt.

Die Reduktion der Verbindungen der Formel II wird daher zweckmäßig in einem sauren oder neutralen Reaktionsmedium durchgeführt. Solche Reaktionsmedien sind jedoch auch für die erfindungsgemäße Reduktion der Verbindungen der Formel I zu Verbindungen der Formel II bevorzugt. Besonders bevorzugt sind saure Reaktionsmedien.

Im Falle der Reduktion der Verbindung der Formel II, worin n Null ist, hat es sich als zweckmäßig erwiesen, einen Phasentransferkatalysator, beispielsweise ein Tetraalkylammoniumsalz, wie Tetramethylammonium-tetrafluoroborat, Tetraäthylammoniumtosylat, Tetraäthylammoniumperchlorat, Tetrabutylammoniumperchlorat, Tetrabutylammonium-tetrafluoroborat, Tetrabutylammoniumbromid, Tetrakis-decylammoniumperchlorat, Hexadecyl-trimethylammoniumbromid oder vorzugsweise Tetrabutylammoniumbromid, dem Reaktionsgemisch zuzusetzen. Die Konzentration des Phasentransferkatalysators beträgt bezogen auf das Gesamtgemisch vorzugsweise etwa 0,05 Mol/l bis etwa 0,8 Mol/l.

Die Konzentration (Gew./Vol.) des Ausgangsmaterials im verwendeten Reaktionsgemisch kann im Falle der Verbindungen der Formeln I und II, worin $n=0$ ist, im allgemeinen zwischen etwa 0,3% und 15%, vorzugsweise etwa 5% und 15% schwanken. Für die Verbindungen der Formeln I und II, worin $n=1$ ist, beträgt sie zweckmäßigerweise etwa 0,3% bis etwa 10% und vorzugsweise etwa 5%.

Die bei der Ausführung des erfindungsgemäßen Verfahrens angewandte Temperatur ist nicht kritisch. Sie wird jedoch nach oben hin durch den Siedepunkt des Reaktionsgemisches begrenzt. Vorzugsweise arbeitet man zwischen Raumtemperatur und etwa 65° C. Das erfindungsgemäße Verfahren kann mit oder ohne Schutzgas durchgeführt werden. Vorzugsweise wird jedoch unter einem Inertgas, beispielsweise Stickstoff oder Argon, gearbeitet.

Das erfindungsgemäße Verfahren kann galvanostatisch oder potentiostatisch durchgeführt werden. Das potentiostatische Verfahren ist bevorzugt.

Das erforderliche Potential ist abhängig vom verwendeten Reaktionsgemisch und Kathodenmaterial und kann durch Messung der Strom-Potential-Kurven (z. B. durch cyclische Voltammetrie) ermittelt werden. Zur Herstellung einer Verbindung der Formel III aus der entsprechenden Verbindung der Formel I muß das Zwischenprodukt der Formel II nicht aus dem Reaktionsgemisch isoliert werden, sondern es kann auch die Verbindung der Formel I durch Anlegen eines entsprechenden negativeren Potentials direkt zur Verbindung der Formel III reduziert werden. Die folgenden Beispiele sollen einen Hinweis auf die Größenordnung der anzuwendenden Potentiale geben:

a) Für die Reduktion der Verbindung der Formel I, worin $n=1$ ist, zur entsprechenden Verbindung der Formel II wird bei Verwendung einer Bleikathode in 10N Schwefelsäure/Dioxan 1 : 1 zweckmäßig ein Potential von etwa —750 mV (gegen gesättigte Kalomelelektrode SCE) und bei Verwendung einer Nickelkathode in 2N Natronlauge ein Potential von etwa —1300 mV (gegen SCE) angewendet.

b) Für die Reduktion der Verbindung der Formel I bzw. II, worin $n=1$ ist, zur entsprechenden Verbindung der Formel III wird bei Verwendung einer Bleikathode in 1N Schwefelsäure/Äthanol 1 : 1 zweckmäßig ein Potential von etwa —1200 mV (gegen SCE) angewendet.

c) Für die Reduktion der Verbindung der Formel I, worin $n=0$ ist, zur entsprechenden Verbindung der Formel II wird bei Verwendung einer Quecksilbersumpfkathode in 3N Schwefelsäure/Dioxan 1 : 1 zweckmäßig ein Potential von etwa —650 mV (gegen gesättigte Silber/Silberchlorid-Elektrode SSE) und in 3N Natronlauge ein Potential von etwa —1500 mV (gegen SCE) angewendet.

d) Für die Reduktion der Verbindung der Formel I bzw. II, worin $n=0$ ist, zur entsprechenden Verbindung der Formel III wird bei Verwendung einer Quecksilbersumpfkathode in 3N Schwefelsäure/Dioxan 1 : 1 und Zusatz von Tetrabutylammoniumhydrogensulfat zweckmäßig ein Potential von etwa —1200 mV (gegen SSE) angewendet.

Die bei der Ausführung des erfindungsgemäßen Verfahrens angewandte Stromdichte ist nicht

kritisch. Im allgemeinen kann sie für die 5-Ring-Verbindungen (n = 0) zwischen etwa 4 und 70 mA/cm$^2$ und für die 6-Ring-Verbindungen (n = 1) zwischen etwa 4 und 40 mA/cm$^2$ schwanken. Bevorzugter Bereich ist etwa 5—15 mA/cm$^2$.

Spannung und Stromstärke sind vom verwendeten Reaktionsmedium, der Größe der Zelle, der angewandten Stromdichte und dergleichen abhängig.

Die Umsetzung der erfindungsgemäß erhaltenen Verbindungen zu Carotinoiden ist dem Fachmann bekannt. Beispielsweise ist die Umsetzung der Verbindungen der Formel III zu Canthaxanthin bzw. Dinorcanthaxanthin in Pure and Appl. Chem. 51, 871—886 beschrieben.

Die Erfindung betrifft ferner alle neuen Verbindungen, Mischungen, Verfahren und Verwendungen wie hierin beschrieben.

Das erfindungsgemäße Verfahren wird durch die folgenden Beispiele weiter veranschaulicht.

## Beispiel 1

Als Reaktionsgefäß dient ein zweiteiliges Glasgefäß (H-Zelle). Der Anodenraum ist vom Kathodenraum durch ein rundes Glassinterdiaphragma (Durchmesser: 3,5 cm) getrennt. Im Kathodenraum befindet sich ein quadratisches Blei-Blech (5 cm × 5 cm) und eine gesättigte Kalomelreferenzelektrode (SCE). Als Anode wird ein Platindraht verwendet. Beide Elektrodenräume sind je mit einem Gaseinleitungsrohr und der Kathodenraum mit einem Magnetrührer versehen. Unter Rühren und Argongaseinleitung werden je 140 ml Dioxan und 10N wäßrige Schwefelsäure in den Kathodenraum sowie je 80 ml Dioxan und 10N wäßrige Schwefelsäure in den Anodenraum gegeben. Nachdem 16,8 g des anfangs nicht vollständig gelösten 2-Hydroxy-3,5,5-trimethyl-2-cyclohexen-1,4-dions in den Kathodenraum gegeben worden sind, wird bei einem konstanten Kathodenpotential von —1150 mV (gegen SCE) unter Rühren und Argongaseinleitung bei Raumtemperatur elektrolysiert, worauf sich eine Stromstärke von ca. 750 mA und eine Zellspannung von ca. 13 V einstellt. Nachdem 41 500 Coulomb, was 108% der theoretisch benötigten Strommenge entspricht, durch die Zelle geflossen sind, wird die Elektrolyse abgebrochen. Die Katholytlösung wird in einen 2-Liter-Scheidetrichter $S_1$ mit 1000 ml Wasser (entionisiert) gegeben. Zwei weitere 2-Liter-Scheidetrichter $S_2$ und $S_3$ werden mit je 500 ml 15%iger Kochsalzlösung beschickt. Durch die drei Scheidetrichter $S_1$—$S_3$ werden der Reihe nach drei Portionen zu je 500 ml Methylenchlorid geschickt. Die organischen Phasen werden über 50 g Natriumsulfat getrocknet und filtriert. Die vereinigten Filtrate werden im Rotationsverdampfer am Wasserstrahlvakuum bei 40° C Badtemperatur bis zur Gewichtskonstanz eingeengt. Man erhält 16,0 g eines weißgelben kristallinen Rückstandes, welcher nach gaschromatographischer Analyse 12,9 g (84%) eines Produkts enthält, welches ein Tautomerengemisch von 3-Hydroxy-2,6,6-trimethyl-2-cyclohexen-1-on und 3-Hydroxy-2,4,4-trimethyl-2-cyclohexen-1-on darstellt. Anschließend wird das Rohprodukt in 45 ml Diisopropyläther heiß gelöst. Nach dem Abkühlen auf Raumtemperatur fallen die ersten weißen Kristalle aus. Danach wird über Nacht bei —20° C stehengelassen, abgenutscht und zweimal mit je 20 ml Diisopropyläther (gekühlt auf —20° C) gewaschen und bei 40° C am Wasserstrahlvakuum bis zur Gewichtskonstanz getrocknet. Man erhält 12,0 g (78%) reines Produkt mit einem Schmelzpunkt von 114—116° C.

## Beispiel 2

a) Als Reaktionsgefäß dient ein zweiteiliges Glasgefäß (H-Zelle). Der Anodenraum ist vom Kathodenraum durch ein rundes Glassinterdiaphragma (Durchmesser: 3,5 cm) getrennt. Im Kathodenraum befindet sich ein quadratisches Blei-Blech (5 cm × 5 cm) und eine gesättigte Kalomelreferenzelektrode (SCE). Als Anode wird ebenfalls ein quadratisches Blei-Blech (3,5 cm × 3,5 cm) verwendet. Beide Elektrodenräume sind je mit einem Gaseinleitungsrohr und der Kathodenraum mit einem Magnetrührer versehen. Unter Rühren und Stickstoffeinleitung werden je 160 ml Dioxan und 10N wäßrige Schwefelsäure in den Kathodenraum sowie je 90 ml Dioxan und 10N wäßrige Schwefelsäure in den Anodenraum gegeben. Nachdem 16,8 g des anfangs nicht vollständig gelösten 2-Hydroxy-3,5,5-trimethyl-2-cyclohexen-1,4-dions in den Kathodenraum gegeben worden sind, wird bei einem konstanten Kathodenpotential von —750 mV (gegen SCE) unter Rühren und Stickstoffeinleitung bei Raumtemperatur elektrolysiert, worauf sich eine Stromstärke von ca. 500 mA einstellt. Nachdem die Stromstärke auf 3 mA abgesunken ist und 19 500 Coulomb, was 101% der theoretisch benötigten Strommenge entspricht, durch die Zelle geflossen sind, wird die Elektrolyse abgebrochen. Die Katholytlösung wird in einen 2-Liter-Scheidetrichter $S_1$ mit 500 ml Wasser (entionisiert) gegeben. Zwei weitere 1-Liter-Scheidetrichter $S_2$ und $S_3$ werden mit je 500 ml 15%ige Kochsalzlösung beschickt. Durch die drei Scheidetrichter $S_1$—$S_3$ werden der Reihe nach neun Portionen zu je 300 ml Methylenchlorid geschickt. Die organischen Phasen werden über 50 g Natriumsulfat getrocknet und filtriert. Die vereinigten Filtrate werden im Rotationsverdampfer am Wasserstrahlvakuum bei 40° C Badtemperatur bis zur Gewichtskonstanz eingeengt. Man erhält 19,1 g eines gelben Harzes, welches nach gaschromatographischer Analyse 15,8 g (93%) eines Produkts enthält, welches ein Tautomerengemisch von 3,4-Dihydroxy-2,6,6-trimethyl-2-cyclohexen-1-on und 3,6-Dihydroxy-2,4,4-trimethyl-2-cyclohexen-1-on darstellt. Das

Rohprodukt wird in 40 ml Diisopropyläther heiß gelöst. Nach dem Abkühlen auf Raumtemperatur fallen die ersten Kristalle aus. Danach wird über Nacht bei —20° C stehen gelassen, abgenutscht und zweimal mit je 20 ml Hexan (gekühlt auf —20° C) gewaschen und bei 50° C am Wasserstrahlvakuum bis zur Gewichtskonstanz getrocknet. Man erhält 15,2 g (89%) reines Produkt mit einem Schmelzpunkt von 107° C.

b) 17,0 g des in Absatz a) erhaltenen Produkts werden für die Weiterreduktion bei einem konstanten Kathodenpotential von —1150 mV (gegen SCE) neu eingesetzt. Die übrigen Versuchsbedingungen erfahren keine Änderung. Nachdem die Stromstärke von anfangs 500 mA auf 30 mA abgesunken ist und 24 300 Coulomb, was 126% der theoretisch benötigten Strommenge entspricht, durch die Zelle geflossen sind, wird die Elektrolyse abgebrochen. Die Katholytlösung wird in einen 2-Liter-Scheidetrichter $S_1$ mit 500 ml Wasser (entionisiert) gegeben. Zwei weitere 1-Liter-Scheidetrichter $S_2$ und $S_3$ werden mit je 500 ml 15%iger Kochsalzlösung beschickt. Durch die drei Scheidetrichter $S_1$—$S_3$ werden der Reihe nach fünf Portionen zu je 300 ml Methylenchlorid geschickt. Die organischen Phasen werden über 50 g Natriumsulfat getrocknet und filtriert. Die vereinigten Filtrate werden im Rotationsverdampfer am Wasserstrahlvakuum bei 40° C Badtemperatur eingeengt. Man erhält 19,3 g eines gelben kristallinen Rückstandes, welcher nach gaschromatographischer Analyse 13,3 g (86%) eines Produktes enthält, welches ein Tautomerengemisch von 3-Hydroxy-2,6,6-trimethyl-2-cyclohexen-1-on und 3-Hydroxy-2,4,4-trimethyl-2-cyclohexen-1-on darstellt. Anschließend wird das Rohprodukt in 35 ml Diisopropyläther heiß gelöst. Nach dem Abkühlen auf Raumtemperatur fallen die ersten weißen Kristalle aus. Danach wird über Nacht bei —20° C stehengelassen, abgenutscht und zweimal mit je 20 ml Hexan (gekühlt auf —20° C) gewaschen und bei 50° C am Wasserstrahlvakuum bis zur Gewichtskonstanz getrocknet. Man erhält 12,8 g (83%) reines Produkt mit einem Schmelzpunkt von 116—117° C.

## Beispiel 3

Als Reaktionsgemäß dient ein zweiteiliges Glasgefäß (H-Zelle). Der Anodenraum ist vom Kathodenraum durch ein rundes Glassinterdiaphragma (Durchmesser: 3,5 cm) getrennt. Im Kathodenraum befindet sich ein quadratisches Nickel-Blech (5 cm x 5 cm) und eine gesättigte Kalomelreferenzelektrode (SCE). Als Anode wird ein quadratisches Bleiblech (3,5 cm x 3,5 cm) verwendet. Beide Elektrodenräume sind je mit einem Gaseinleitungsrohr und der Kathodenraum mit einem Magnetrührer versehen. Unter Rühren und Stickstoffeinleitung werden 320 ml 2N wäßrige Natronlauge und 16,8 g 2-Hydroxy-3,5,5-trimethyl-2-cyclohexen-1,4-dion in den Kathodenraum sowie 180 ml 2N wäßrige Natronlauge in den Anodenraum gegeben und anschließend bei einem konstanten Kathodenpotential von —1300 mV (gegen SCE) bei Raumtemperatur elektrolysiert. Nachdem die Stromstärke von 300 mA auf 10 mA abgesunken ist und 21 200 Coulomb, was 110% der theoretisch benötigten Strommenge entspricht, durch die Zelle geflossen sind, wird die Elektrolyse abgebrochen. Die Katholytlösung wird in einem 2-Liter-Scheidetrichter $S_1$ mit 500 ml 3N wäßriger Schwefelsäure sauer gestellt. Zwei weitere Scheidetrichter $S_2$ und $S_3$ werden mit je 400 ml 15%iger Kochsalzlösung beschickt. Durch die drei Scheidetrichter $S_1$—$S_3$ werden der Reihe nach neun Portionen zu je 200 ml Methylenchlorid geschickt. Die organischen Phasen werden über 50 g Natriumsulfat getrocknet und filtriert. Die vereinigten Filtrate werden im Rotationsverdampfer am Wasserstrahlvakuum bei 40° C Badtemperatur bis zur Gewichtskonstanz eingeengt. Man erhält 17,0 g eines rosaroten kristallinen Rückstandes, welcher nach gaschromatographischer Analyse 13,1 g (77%) eines Produktes enthält, welches ein Tautomerengemisch von 3,4-Dihydroxy-2,6,6-trimethyl-2-cyclohexen-1-on und 3,6-Dihydroxy-2,4,4-trimethyl-2-cyclohexen-1-on darstellt. Das Rohprodukt wird in 40 ml Diisopropyläther heiß gelöst. Nach dem Abkühlen auf Raumtemperatur fallen die ersten Kristalle aus. Danach wird über Nacht bei —20° C stehen gelassen, abgenutscht und zweimal mit je 20 ml Hexan (gekühlt auf —20° C) gewaschen und bei 40° C am Wasserstrahlvakuum bis zur Gewichtskonstanz getrocknet. Man erhält 12,7 g (75%) reines Produkt mit einem Schmelzpunkt von 104—105° C.

## Beispiel 4

Als Reaktionsgefäß dient ein zweiteiliges, thermostatisierbares Glasgefäß (H-Zelle). Der Anodenraum ist vom Kathodenraum durch eine auswechselbare NAFION®-Membran (Dupont) mit 4 cm Durchmesser getrennt. Im Kathodenraum befindet sich eine Quecksilbersumpfelektrode (Oberfläche: ca. 12,5 $cm^2$). Als Anode wird ein Platinblech (2,5 cm x 2,5 cm) verwendet. Beide Elektrodenräume sind je mit einem Gaseinleitungsrohr und der Kathodenraum mit einem Magnetrührer und Thermometer versehen. Unter Rühren und Argongaseinleitung werden je 50 ml Dioxan und 3N wäßrige Schwefelsäure sowie 3,4 g Tetrabutylammoniumhydrogensulfat und 15,0 g 4-Hydroxy-2,2,5-trimethyl-4-cyclopenten-1,3-dion in den Kathodenraum und je 50 ml Dioxan und 3N wäßrige Schwefelsäure in den Anodenraum gegeben. Sobald die Reaktionslösung auf eine konstante Temperatur von 65° C gebracht worden ist, wird bei einer konstanten Stromstärke von 100 mA unter Rühren und Argonbegasung elektrolysiert, wobei sich eine Zellspannung von ca. 3,5 V einstellt. Nachdem 44 600 Coulomb, was

119% der theoretisch benötigten Strommenge entspricht, durch die Zelle geflossen sind, wird die Elektrolyse abgebrochen. Die Katholytlösung wird in einen 500-ml-Scheidetrichter $S_1$ mit 200 ml 15%iger Kochsalzlösung gegeben. Zwei weitere 500-ml-Scheidetrichter $S_2$ und $S_3$ werden mit je 400 ml 15%iger Kochsalzlösung beschickt. Durch die drei Scheidetrichter $S_1$—$S_3$ werden der Reihe nach neun Portionen zu je 150 ml Methylenchlorid geschickt. Die organischen Phasen werden über 20 g Natriumsulfat getrocknet und filtriert. Die vereinigten Filtrate werden im Rotationsverdampfer am Wasserstrahlvakuum bei 40° C Badtemperatur bis zur Gewichtskonstanz eingeengt. Man erhält 21,3 g eines gelblichen, kristallinen Rückstandes, welcher auf einer mit 100 g Kieselgel beschickten Chromatographiersäule gereinigt wird. Als Laufmittel werden 5 l Diäthyläther verwendet. Nach Vereinigung der reinen Fraktionen wird am Wasserstrahlvakuum bei 40° C Badtemperatur bis zur Gewichtskonstanz eingeengt. Man erhält 11,0 g eines gelblichen, kristallinen Rückstandes, welcher in einem Gemisch bestehend aus je 50 ml Diisopropyläther und Aceton heiß gelöst wird. Nach dem Abkühlen auf Raumtemperatur fallen die ersten weißen Kristalle aus. Danach wird über Nacht bei —20° C stehengelassen, abgenutscht und zweimal mit je 20 ml Diisopropyläther (gekühlt auf —20° C) gewaschen und bei 50° C am Wasserstrahlvakuum bis zur Gewichtskonstanz getrocknet. Man erhält 9,6 g (70%) eines Produkts mit einem Schmelzpunkt von 170° C, welches ein Tautomerengemisch von 3-Hydroxy-2,5,5-trimethyl-2-cyclopenten-1-on und 3-Hydroxy-2,4,4-trimethyl-2-cyclopenten-1-on darstellt.

## Beispiel 5

a) Als Reaktionsgefäß dient ein zweiteiliges Glasgefäß (H-Zelle). Der Anodenraum ist vom Kathodenraum durch ein rundes Glassinterdiaphragma (Durchmesser: 3,5 cm) getrennt. Im Kathodenraum befindet sich eine Quecksilbersumpfelektrode (Oberfläche: ca. 28 $cm^2$) und eine gesättigte Silber/Silberchlorid-Referenzelektrode (SSE). Als Anode wird ein Blei-Blech (3,5 cm × 3,5 cm) verwendet. Beide Elektrodenräume sind mit je einem Gaseinleitungsrohr und der Kathodenraum mit einem Magnetrührer versehen. Unter Rühren und Stickstoffgaseinleitung werden je 160 ml Dioxan und 3N wäßrige Schwefelsäure in den Kathodenraum sowie je 90 ml Dioxan und 3N wäßrige Schwefelsäure in den Anodenraum gegeben. Nachdem 15,4 g des anfangs nicht vollständig gelösten 4-Hydroxy-2,2,5-trimethyl-4-cyclopenten-1,3-dion in den Kathodenraum gegeben worden sind, wird bei einem Kathodenpotential von —650 mV (gegen SSE) unter Rühren und Stickstoffeinleitung bei Raumtemperatur elektrolysiert, worauf sich eine Stromstärke von ca. 200 mA einstellt. Nachdem die Stromstärke auf 5 mA abgesunken ist und 19 300 Coulomb, was 100% der theoretisch benötigten Strommenge entspricht, durch die Zelle geflossen sind, wird die Elektrolyse abgebrochen. Die vereinigten Katholyt- und Anolytlösungen werden in einen 2-Liter-Scheidetrichter $S_1$ mit 500 ml gesättigter Kochsalzlösung gegeben. Zwei weitere 1-Liter-Scheidetrichter $S_2$ und $S_3$ werden mit je 200 ml Kochsalzlösung beschickt. Durch die drei Scheidetrichter $S_1$—$S_3$ werden der Reihe nach eine Portion zu 300 ml und neun Portionen zu 100 ml Methylenchlorid geschickt. Die organischen Phasen werden über 50 g Natriumsulfat getrocknet und filtriert. Die vereinigten Filtrate werden im Rotationsverdampfer am Wasserstrahlvakuum bei 40° C Badtemperatur bis zur Gewichtskonstanz eingeengt. Man erhält 17,6 g weiße Kristalle. Das Rohprodukt wird in einem Gemisch, bestehend aus je 50 ml Diisopropyläther und Aceton, heiß gelöst. Nach dem Abkühlen auf Raumtemperatur fallen die ersten weißen Kristalle aus. Danach wird über Nacht bei —20° C stehengelassen, abgenutscht und zweimal mit je 20 ml Diisopropyläther (gekühlt auf —20° C) gewaschen und bei 50° C am Wasserstrahlvakuum bis zur Gewichtskonstanz getrocknet. Man erhält 9,5 g (61%) eines Produkts mit einem Schmelzpunkt von 147—148° C, welches ein Tautomerengemisch von 3,4-Dihydroxy-2,5,5-trimethyl-2-cyclopenten-1-on und 3,5-Dihydroxy-2,4,4-trimethyl-2-cyclopenten-1-on darstellt.

b) Neben je 45 ml Dioxan und 3N wäßriger Schwefelsäure sowie 3,05 g Tetrabutylammoniumhydrogensulfat werden 15,6 g des in Absatz a) erhaltenen Produkts für die Weiterreduktion bei einem Kathodenpotential von —1200 mV (gegen SSE) in den Kathodenraum gegeben. Der Anodenraum, versehen mit je 50 ml Dioxan und 3N wäßriger Schwefelsäure sowie einer Platindraht-Anode, ist vom Kathodenraum durch eine runde Polymermembran (Durchmesser: 4 cm) getrennt. Die übrigen Versuchsbedingungen erfahren keine Änderung. Nachdem die Stromstärke von anfangs 150 mA auf 70 mA abgesunken ist und 27 100 Coulomb, was 140% der theoretisch benötigten Strommenge entspricht, durch die Zelle geflossen sind, wird die Elektrolyse abgebrochen. Die Katholytlösung wird in einem 500-ml-Scheidetrichter $S_1$ mit 150 ml gesättigter Kochsalzlösung gegeben. Zwei weitere 500-ml-Scheidetrichter $S_2$ und $S_3$ werden mit je 150 ml gesättigter Kochsalzlösung beschickt. Durch die drei Scheidetrichter $S_1$—$S_3$ werden der Reihe nach neun Portionen zu je 150 ml Methylenchlorid geschickt. Die organischen Phasen werden über 50 g Natriumsulfat getrocknet und filtriert. Die vereinigten Filtrate werden im Rotationsverdampfer am Wasserstrahlvakuum bei 40° C Badtemperatur bis zur Gewichtskonstanz eingeengt. Man erhält 14,9 g eines gelben kristallinen Rückstandes, welcher nach Reinigung (Abtrennen des polaren Tetrabutylammonium-hydrogensulfats) auf einer Chromatographiersäule (Kieselgel/Diäthyläther) 11,9 g weiß-gelbe Kristalle ergab. Nach der Umkristallisation aus einem Gemisch, bestehend aus je 50 ml Diisopropyläther und Aceton, werden 9,9 g (71%) eines Produkts mit einem Schmelzpunkt von 169—179° C erhalten, welches ein Tautomerengemisch von

3-Hydroxy-2,5,5-trimethyl-2-cyclopenten-1-on und 3-Hydroxy-2,4,4-trimethyl-2-cyclopenten-1-on darstellt.

Beispiel 6

Als Reaktionsgefäß dient ein zweiteiliges Glasgefäß (H-Zelle). Der Anodenraum ist vom Kathodenraum durch ein rundes Glassinterdiaphragma (Durchmesser: 2,5 cm) getrennt. Im Kathodenraum befindet sich eine Graphitelektrode (3,5 cm x 3,5 cm) und eine gesättigte Silber/Silberchlorid-Referenzelektrode (SSE). Als Anode wird eine mit Bleidioxid beschichtete Titananode verwendet. Beide Elektrodenräume sind je mit einem Gaseinleitungsrohr und der Kathodenraum zudem mit einem Magnetrührer versehen. Unter Rühren und Argongaseinleitung werden je 35 ml Äthanol und 1N wäßrige Schwefelsäure in den Kathodenraum sowie je 35 ml Äthanol und 1N wäßrige Schwefelsäure in den Anodenraum gegeben. Nachdem 0,7 g 2-Hydroxy-3,5,5-trimethyl-2-cyclohexen-1,4-dion in den Kathodenraum gegeben worden sind, wird bei einem konstanten Kathodenpotential von —1250 mV (gegen SSE) unter Rühren und Argongaseinleitung bei Raumtemperatur elektrolysiert, worauf sich eine Stromstärke von ca. 306 mA einstellt. Nachdem 2600 Coulomb, was 162% der theoretisch benötigten Strommenge entspricht, durch die Zelle geflossen sind, wird die Elektrolyse abgebrochen. Die Katholytlösung wird fünfmal mit je 50 ml Chloroform extrahiert. Die vereinigten Extrakte werden über Natriumsulfat getrocknet, filtriert und eingeengt. Die eingeengte Lösung enthält gemäß gaschromatographischer Analyse 0,45 g (70%) des Tautomerengemisches von 3-Hydroxy-2,6,6-trimethyl-2-cyclohexen-1-on und 3-Hydroxy-2,4,4-trimethyl-2-cyclohexen-1-on und 0,022 g (3%) des Tautomerengemisches von 3,4-Dihydroxy-2,6,6-trimethyl-2-cyclohexen-1-on und 3,6-Dihydroxy-2,4,4-trimethyl-2-cyclohexen-1-on.

**Patentansprüche**

1. Verfahren zur Herstellung von 3-Hydroxy-2-cycloalken-1-on-Derivaten, dadurch gekennzeichnet, daß man eine Verbindung der allgemeinen Formel

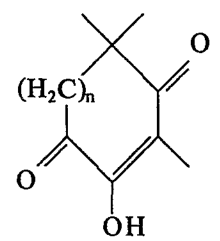

(I)

worin n die Zahlen 0 oder 1 bedeutet,
zu einer Verbindung der allgemeinen Formel

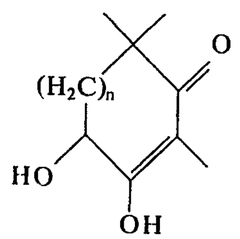

(II)

worin n die obige Bedeutung hat,
kathodisch reduziert und, gewünschtenfalls, die erhaltene Verbindung in saurem oder neutralem Medium zu einer Verbindung der allgemeinen Formel

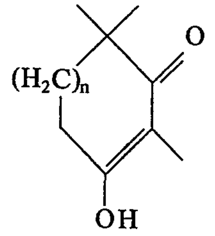

(III)

worin n die obige Bedeutung hat,
kathodisch weiterreduziert.

2. Verfahren nach Anspruch 1, dadurch gekennzeichnet, daß man die Reduktion der Verbindung der Formel II, worin n die Zahl 0 ist, unter Zusatz eines Phasentransferkatalysators durchführt.

3. Verfahren nach Anspruch 1 oder 2, dadurch gekennzeichnet, daß man die Reduktion in einer unterteilten Zelle durchführt.

4. Verfahren nach einem der Ansprüche 1 bis 3, dadurch gekennzeichnet, daß man die Reduktion in einem sauren Reaktionsmedium durchführt.

5. Verfahren nach Anspruch 4, dadurch gekennzeichnet, daß man die Reduktion in einem Gemisch einer wäßrigen Mineralsäure und eines inerten organischen Lösungsmittels durchführt.

6. Verfahren nach Anspruch 5, dadurch gekennzeichnet, daß man als Mineralsäure Schwefelsäure verwendet.

7. Verfahren nach Anspruch 5 oder 6, dadurch gekennzeichnet, daß man als inertes organisches Lösungsmittel Methanol, Äthanol, t-Butanol, Tetrahydrofuran oder 1,4-Dioxan verwendet.

8. Verfahren nach einem der Ansprüche 1 bis 7, dadurch gekennzeichnet, daß man als Kathodenmaterial Blei, Graphit oder Quecksilber verwendet.

9. Verfahren nach einem der Ansprüche 1 bis 8, dadurch gekennzeichnet, daß man die Reduktion bei einer Temperatur zwischen Raumtemperatur und der Siedetemperatur des Reaktionsgemisches durchführt.

10. Verbindung der Formel

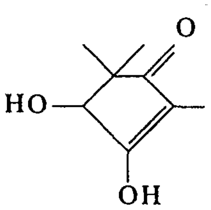

(IIa)

**Claims**

1. A process for the manufacture of 3-hydroxy-2-cycloalken-1-one derivatives, characterized by cathodically reducing a compound of the general formula

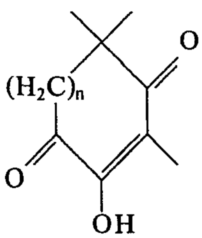

(I)

wherein n signifies the numbers 0 or 1,
to a compound of the general formula

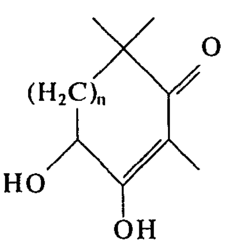

(II)

wherein n has the above significance,
and, if desired, further cathodically reducing the resulting compound in an acidic or neutral medium to a compound of the general formula

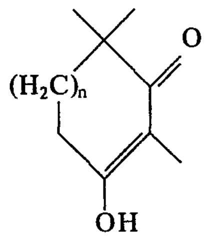

(III)

wherein n has the above significance.

2. A process according to claim 1, characterized in that the reduction of the compound of formula II, wherein n is the number 0, is carried out with the addition of a phase transfer catalyst.

3. A process according to claim 1 or 2, characterized in that the reduction is carried out in a sub-divided cell.

4. A process according to any one of claims 1 to 3, characterized in that the reduction is carried out in an acidic reaction medium.

5. A process according to claim 4, characterized in that the reduction is carried out in a mixture of an aqueous mineral acid and an inert organic solvent.

6. A process according to claim 5, characterized in that sulphuric acid is used as the mineral acid.

7. A process according to claim 5 or 6, characterized in that methanol, ethanol, t-butanol, tetrahydrofuran or 1,4-dioxane is used as the inert organic solvent.

8. A process according to any one of claims 1 to 7, characterized in that lead, graphite or mercury is used as the cathode material.

9. A process according to any one of claims 1 to 8, characterized in that the reduction is carried out at a temperature between room temperature and the boiling temperature of the reaction mixture.

10. The compound of the formula

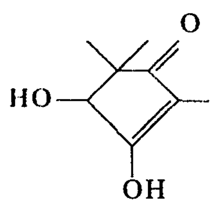

(IIa)

**Revendications**

1. Procédé de préparation de dérivés de 3-hydroxy-2-cycloalcén-1-one, caractérisé en ce qu'on réduit un composé de formule générale

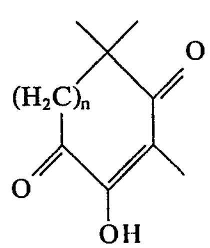

(I)

où n représente les nombres 0 ou 1, en un composé de formule générale

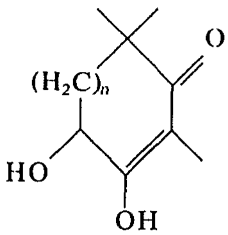

(II)

où n a la signification donnée ci-dessus, de façon cathodique et, si on le désire, en ce qu'on réduit plus avant de façon cathodique le composé obtenu en milieu acide ou neutre en un composé de formule générale

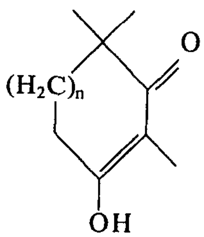

(III)

où n a la signification donnée ci-dessus.

2. Procédé selon la revendication 1, caractérisé en ce qu'on conduit la réduction du composé de formule II où n est le nombre 0 avec addition d'un catalyseur de transfert de phase.

3. Procédé selon les revendications 1 et 2, caractérisé en ce qu'on conduit la réduction dans une cellule divisée.

4. Procédé selon l'une des revendications 1 à 3, caractérisé en ce qu'on conduit la réduction dans un milieu réactionnel acide.

5. Procédé selon la revendication 4, caractérisé en ce qu'on conduit la réduction dans un mélange d'un acide minéral aqueux et d'un solvant organique inerte.

6. Procédé selon la revendication 5, caractérisé en ce qu'on utilise comme acide minéral l'acide sulfurique.

7. Procédé selon les revendications 5 et 6, caractérisé en ce qu'on utilise comme solvant organique inerte le méthanol, l'éthanol, le t-butanol, le tétrahydrofuranne ou le 1,4-dioxanne.

8. Procédé selon l'une des revendications 1 à 7, caractérisé en ce qu'on utilise comme matière de cathode le plomb, le graphite ou le mercure.

9. Procédé selon l'une des revendications 1 à 8, caractérisé en ce qu'on conduit la réduction à une température située entre la température ambiante et la température d'ébullition du mélange réactionnel.

10. Composé de formule

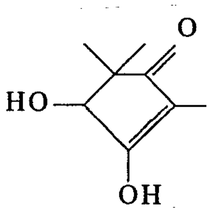

(IIa)